# EUROPÄISCHE PATENTANMELDUNG

(11) **EP 4 516 115 A2**
(43) Veröffentlichungstag der Anmeldung: **05.03.2025**
(21) Anmeldenummer: 24218693.0
(22) Anmeldetag: 12.11.2015
(51) Int. Cl.: A23L 2/40, A23L 33/16, A61K 9/46

(54) **VERFAHREN ZUR HERSTELLEN EINER NATRIUM-ION- FREIEN BRAUSETABLETTE ODER EINES SOLCHEN BRAUSEPULVERS ODER GRANULATS MIT HOHEM X-ION-GEHALT, WOBEI X VERSCHIEDENE STOFFE SEIN KÖNNEN**

(30) Priorität: 12.11.2014 CH 17582014
(62) Teilanmeldung aus: 15820259.8
(71) Anmelder: Biofar Laboratoires Sàrl, 92752 Nanterre Cedex (FR) (FR)
(72) Erfinder: STROBEL, Hanspeter, verstorben (CH)
(74) Vertreter: Felber, Josef

(57) **Zusammenfassung**

Das Verfahren dient zum Herstellen von Natrium-lon-freien X-Carbonat oder Hydrocarbonat Brausepulver, -granulat oder Brausetabletten. Die Wirksubstanzen X, wenigstens Calcium-Carbonat, mit wenig Alkohol und Wasser gefeuchtet, werden als Pulver in einem Vakuumkessel bei ständigem Abpumpen in einem Teilvakuum mit wenig Lactobionsäure vermischt. Dadurch reagiert das Calciumsalz (CaCOs) des Calciums in der Oberflächenschicht der Pulverpartikel mit der Lactobionsäure, verbraust und setzt wenig CO₂ frei. In der Folge steigt der Druck im Vakuumkessel an und nach Ende der Brause-Reaktion sinkt der Innendruck im Vakuumkessel durch das ständige Abpumpen wieder auf den Anfangswert ab. Dabei trocknet das Pulver aus. Nach Entnahme aus dem Vakuumtopf wird eine Säure, vorzugsweise Zitronensäure als Brausemittel zugegeben und das Pulver wird hernach wahlweise zu Tabletten verpresst. Die so erzeugte Natrium-lon-freie Calcium-Carbonat Brausetablette enthält daher wenigstens Calcium-Carbonat sowie Zitronensäure als Brausemittel, wobei die verpressten Partikel mittels Lactobionsäure in ihrer Oberflächenschicht angebraust sind.

## Beschreibung

Diese Erfindung betrifft ein Verfahren zum Herstellen einer Natrium-Ion-freien Brausetablette oder eines solchen Brausepulvers oder -granulats als Lieferant von wahlweise Calcium, Magnesium, Kalium oder einer Mischung aus diesen Stoffen, wobei diese Brausetablette bzw. dieses Brausepulver oder -granulat rasch und vollständig in Wasser löslich sein soll.

Zum allgemeinen Verbrauch hergestellte Nahrungsmittelprodukte werden oft durch Nährstoffzusätze oder andere Arten von Zusatzstoffen verändert, um ihre Nährstoffeigenschaften zu verbessern. Die Nährstoffverstärkung von Nahrungsmittelprodukten kann Zusatzstoffe beinhalten, die für den gesamten Gesundheitszustand des menschlichen Körpers förderlich sind. Beispiele der Nährstoffverstärkung sind die Zugabe von Vitaminen, Mineralien und vergleichbaren Materialien. Diese Zusatzstoffe sind für den menschlichen Stoffwechsel entweder absolut notwendig oder sie fördern die Bereitstellung von Substanzen, die bei einer normalen Ernährung nicht in genügenden Mengen verfügbar sind.

In der jüngsten Vergangenheit hat die Calciumverstärkung von Nahrungsmitteln und Getränken eine bedeutende Beachtung erlangt. Eine Calciumverstärkung und erhöhte Calciumaufnahme sind nach Berichten besonders nützlich für die Verhinderung oder Milderung der Wirkungen von Osteoporose. Eine erhöhte diätetische Calciumaufnahme hat sich für die Minimierung von Knochenverlust bei Erwachsenen und älteren Personen als wirksam erwiesen. Ausserdem kann erhöhter Calciumverzehr in jüngeren Jahren Reserven schaffen, die in späteren Jahren eine grössere Toleranz bei einer negativen Calciumbilanz ermöglichen. Erhöhter Calciumverbrauch soll ohne Rücksicht auf das Alter die Wirkungen der Osteoporose mässigen oder verzögern. Daher könnten Personen jeden Alters von einem erhöhten Calciumverbrauch Vorteile haben. Leider verbrauchen viele der Personen mit dem stärksten Calciumbedarf, darunter Kinder, Frauen und ältere Leute nicht die empfohlenen täglichen Calciummengen. Nach Übersichten des United States Department of Agriculture verbrauchen z. B. neun von zehn Frauen in den USA nicht die empfohlenen Calciummengen. Die älteren Leute haben wegen abnehmenden Appetits und Stoffwechsels oft Schwierigkeiten, ihren Calciumverbrauch zu erhöhen. Neben der Knochengesundheit weist die jüngste Forschung auf die Wichtigkeit von Calcium zur Verbesserung der Dickdarmgesundheit, der Gewichtskontrolle und anderer Gesundheitskriterien hin. Der gegenwärtig in Fachkreisen als ideal empfohlene Tageswert für die Calciumzufuhr liegt bei 1000 mg pro Tag.

Diese Calciumergänzungsstoffe wurden in einer grossen Vielzahl von Nahrungsmittelprodukten eingesetzt. US-Patent 4,784,871 (15. November 1988) sieht z. B. einen mit Calcium verstärkten Joghurt vor. Nach dem Patent könnte irgendeine säurelösliche Calciumverbindung verwendet werden. US-Patent 5,449,523 (12. September 1995) und US-Patent 5,820,903 (13. Oktober 1998) sehen ebenfalls mit Calcium angereicherte Joghurts vor. US-Patent 5,478,587 (27. Dezember 1995) gibt mit Calcium angereicherte Desserts an.

US-Patent 5,834,045 (10. November 1998) gibt mit Calcium verstärkte saure Getränke an. Dieses Patent berichtet, dass der Einsatz einer Calciumhydroxid und Calciumglycerophosphat enthaltenden Calciumquelle mit einem Ansäuerungsmittel ein Getränkeprodukt mit einer deutlichen Verbesserung der Lagerbeständigkeit ergibt. US-Patent 5,855,936 (5. Januar 1999) beschreibt eine Mischung von Calciumsalzen im Gleichgewicht mit löslichen und unlöslichen Salzen, die mit einer Glucuronsäurequelle stabilisiert sind. Diese Zusammensetzung kann Milchgetränke und andere Produkte auf Milchbasis ohne Koagulierung und Sedimentierung und mit verbesserter Schmackhaftigkeit verstärken. Die Calciumsalze müssen mit der Glucuronsäurequelle stabilisiert sein. Andere Calciumquellen könnten wahlweise enthalten sein. Andere mit Calcium angereicherte Getränke sind z B. beschrieben in US-Patent 4,642,238 (10. Februar 1987, diätetisch und ernährungsmässig ausgeglichene Getränke); US-Patent 4,701,329 (20. Oktober 1987, Milch); US-Patent 4,737,375 (12. April 1988, carbonisierte und nicht carbonisierte Getränke mit löslich gemachtem Calcium und spezifischen Mengen.

Herkömmlich gibt es auch Brausetabletten zum Zuführen von Calcium als ergänzenden Zusatz für den Bedarf des menschlichen und auch tierischen Körpers. Diese Calcium-Brausetabletten sind seit vielen Jahrzehnten bekannt, doch greifen sie stets auf Natrium-Ion (HST) zurück, um die Löslichkeit in Wasser sicherzustellen. Natrium hat die negative Wirkung, dass es den Blutdruck erhöht, weil es das Wasser im Blut zurückhält, was gerade bei älteren Menschen mit langsamerer Verdauung kritisch sein kann und das Risiko eines Schlaganfalls oder einer Trombose erhöht. Die Brausetabletten sollten daher am besten kein Natrium-Ion bzw. kein Natron enthalten.

Diese Calcium-Ion-Brausetabletten (CaCos) werden bei Calcium- und Vitamin-D-Mangel verabreicht oder auch eingesetzt, um Harnsteine, wie Nierensteine, Blasensteine oder Harnleitersteine, das heisst Ablagerungen von Harnsalzen zu bekämpfen und zu lösen. Die in Blemaren enthaltenen Zitrate alkalisieren den Harn, das heisst sie erhöhen den pH-Wert des Urins. Dadurch können Steine aufgelöst werden, bzw. am Wachstum gehindert werden.

Eine typische Zusammensetzung einer solchen Brausetablette enthält zum Beispiel, in Bezug auf Calzium und Natrium-Ion:

| | | |
|---|---|---|
| Calciumcarbonat | 2'000 | mg |
| Calcium-Ion | 800 | mg |
| Natriumhydrogencarbonat | 520 | mg |
| Natrium-Ion (HST) | 145 | mg |
| Zitronensäure | 3`700 | mg |
| Cholecalciferol | 0.02 | mg |
| entspricht | 800 | I.E. (Internationale Einheiten) |

sowie weitere Inhaltsstoffe, bei einem Gesamtgewicht von 6`500 mg.

Aufgabe dieser Erfindung ist es vor diesem Hintergrund, ein Verfahren aufzuzeigen, wie eine X Ion-Brausetablette bzw. ein solches Brausepulver oder - granulat hergestellt werden kann, ohne Natron auskommend, und trotzdem in Wasser durch Brausen rasch und vollständig lösbar, sowie die hierzu nötigen Bestandteile der Brausetablette bzw. des Brausepulvers anzugeben. Als X soll dabei insbesondere Calcium zur Herstellung von Calcium-Ion-Brausetabletten (CaCos) eingesetzt werden, wobei das Verfahren aber auch mit Magnesium oder Kalium oder einer Mischung aus diesen Stoffen anstelle von Calcium bzw. X durchführbar sein soll.

Die Aufgabe wird gelöst durch ein Verfahren mit den Merkmalen des Anspruchs 1.

Weiter wird die Aufgabe gelöst durch nach diesem Verfahren hergestellte Natrium-Ion-freie Brausetablette bzw. eines solchen Brausepulvers oder -granulats, gekennzeichnet durch die Merkmale des Anspruchs 5.

Als Beispiel einer Zusammensetzung einer Brausetablette mit Calciumcarbonat als Hauptbestandteil dient die folgende Zusammensetzung:

| | | |
|---|---|---|
| Calciumcarbonat | 2'000 | mg |
| Kaliumhydrogencarbonat | 550 | mg |
| Lactobionsäure | 480 | mg |
| Zitronensäure | 3'200 | mg |
| Cholecalciferol | 0.02 | mg |
| entspricht | 800 | I.E. (Internationale Einheiten) |

Calciumcarbonat ist eigentlich nichts anderes als Marmor, das als sehr feines Pulver vorliegt. Und dieses Pulver löst sich im Wasser praktisch nicht auf, nämlich bloss 0.014 Gramm Calciumcarbonat lassen sich in einem Liter Wasser lösen. Mit CO₂ Sprudel im Wasser aber lassen sich bis zu 16.6 Gramm Calciumcarbonat in einem Liter Wasser lösen. Es gilt daher, im Wasser zunächst das Gas CO₂ zu erzeugen. Und genau dazu lässt sich zum Beispiel Kaliumhydrogencarbonat hervorragend einsetzen, denn in kleinen Mengen gilt Kaliumhydrogencarbonat als Nahrungsmittelzusatz und kann daher bedenkenlos hierzu verwendet werden. Auf 3000 mg Calciumcarbonat (auch Magnesiumcarbonat, Magnesiumhydrogencarbonat, Kaliumcarbonat und Kaliumhydrogencarbonat je alleine oder Mischungen davon mit Calciumcarbonat erzeugen die gleiche Effekte) reichen 550mg Kaliumhydrogencarbonat, welches mit Lactobionsäure zum Brausen und damit und zum Erzeugen von CO₂ gebracht wird. Das entstandene CO₂ seinerseits bewirkt dann eine entscheidende Verbesserung der Wasserlöslichkeit des Calciumcarbonates, nämlich bis zu 16.6 Gramm pro Liter Wasser. Gleichzeitig sorgt auch das Lactobionat-Ion für eine bessere Löslichkeit der Calcium-Ionen. Kaliumhydrogencarbonat wird im Prinzip in Zusammenwirkung mit der Lactobionsäure als Starter für den Löslichkeitsprozess für das Calciumcarbonat eingesetzt.

Weiter wird durch das Anbrausen der Calciumcarbonatpartikel mittels Lactobionsäure eine unregelmässige Zersetzung deren Oberflächenschicht bewirkt, in dem dort einzelne Carbonat-Ionen mit der Lactobionsäure regieren und CO₂ freisetzen. Das macht die Oberflächen der Partikel porös und rau. Dann trocknet man das zuvor mit Alkohol und Wasser (= Granulierflüssigkeit) angefeuchtete Pulver im Teilvakuum aus. Dieser Prozess kann nötigenfalls wiederholt werden, um eine innige Anbrausung zu bewirken. Das so vorpräparierte Pulver wird mit einer Säure als Brausemittel versetzt, vorzugsweise mit Zitronensäure, und hernach zu Tabletten verpresst. Anstelle von Zitronensäure kann auch Apfelsäure, Bernsteinsäure, Fumarsäure, Weinsäure oder Ascorbinsäure verwendet werden, oder Mischungen aus allen diesen Säuren.

Die so erzeugten Calcium-Ion-Brausetabletten (CaCos) dienen zur mineralischen Verstärkung von Nahrungsmittel- und Getränkeprodukten. Sie bilden eine stabile, rein schmeckende Calciumquelle, die zur Calciumverstärkung sehr verschiedener Nahrungsmittel- und Getränkeprodukte geeignet ist. Wenn anstelle von Calcium andere Stoffe treten, wie etwa Magnesium oder Kalium oder auch Mischungen all dieser Stoffe, so werden entsprechend weitere Ergänzungsstoffe für den Körper verfügbar gemacht.

Lactobionsäure ist im Allgemeinen ein weisses kristallines Pulver. Lactobionsäure kann im Handel bezogen werden (z. B. Fairlawn, N.J.; Sigma, St. Louis, MO) oder durch chemische oder enzymatische Oxidation von Lactose oder eines Lactose enthaltenden Substrats hergestellt werden. Die Lactobionsäure kann durch chemische Saccharid-Oxidation oder Bioumsetzungsverfahren (zum Beispiel katalytische Wirkung eines Kohlenhydratoxidase-Enzyms) unter Verwendung von Lactose oder eines Lactose enthaltenden Substrats (zum Beispiel Molke oder Molke-Permeat) als Ausgangsmaterial hergestellt werden. Im Prinzip handelt es sich bei der Lactobionsäure um ein Nebenprodukt der Milchindustrie. Geeignete Kohlenhydratoxidase-Enzyme sind zum Beispiel Lactoseoxidase, Glucoseoxidase, Hexoseoxidase und dergleichen sowie Gemische daraus. Im Allgemeinen wird Lactoseoxidase bevorzugt. Ein besonders geeignetes Enzym zur Lactoseoxidation wurde von Novozymes NS entwickelt und ist in WO 99/31990 beschrieben.

Das erfindungsgemässe Verfahren erlaubt die Herstellung von Brausetabletten bzw. Brausegranulaten, welche nicht nur eine ausgezeichnete Lagerungsstabilität besitzen, sondern auch in ihrer Qualität immer gleichbleibend sind, da die Reproduzierbarkeit des Verfahrens durch Kontrolle einer Reihe von Reaktionsparametern jederzeit gewährleistet ist. Das Verfahren zur Herstellung von Brausetabletten oder verarbeitbaren Brausegranulaten erfolgt durch Wärmebehandlung bei 30°C bis 100°C aus Säure und Hydrogencarbonat und/oder Carbonat zusammen mit der Granulierflüssigkeit als wesentliche Brausebestandteile. Das pulverförmige oder körnige Gemisch wird in einem geschlossenen System im Teilvakuum behandelt, wobei die Säure (eine dosierte Menge von bis zu 7 Masse-%, bezogen auf die Masse des Gemisches, Lactobionsäure) mit der nötigen Menge an Hydrogencarbonat und/oder Carbonat vermischt wird und man hierauf dieses Gemisch mit einem polaren Lösungsmittel wie Wasser, Alkohol, Methanol, oder Mischungen hiervon versetzt und granuliert wird. Die Vakuumbehandlung bei einer Temperatur von 30°C bis 100°C, vorzugsweise 40°C bis 80°C, führt zur Aufrauhung der Oberflächenschicht von zumindest einer der Reaktionskomponenten bzw. zur Überführung derselben in einen trockenen Zustand mit schnellerer Löslichkeit. Eine dadurch aufgrund der sich einstellenden Reaktion entstehende CO₂-Entwicklung lässt den Druck im Vakuumkessel zunächst ansteigen, bis max. 1000 mbar (Atmosphärendruck). Aus der Druckdifferenz zum Anfangswert von zum Beispiel 10mbar im Vakuumkessel lässt sich das Volumen und die Masse des freigesetzten CO₂ ermitteln. Die Wärmebehandlung jeweils nach der Vakuumschnelltrocknung des Gemisches kann sooft wiederholt werden, bis die durch deutliche Verlangsamung der Reaktion bzw. verminderte Gasentwicklung das Ende der Oberflächen-Aufrauhung anzeigt.

Die erhaltenen Aggregate werden hernach zu einer gewünschten Teilchengrösse zerkleinert, gegebenenfalls mit den gewünschten Zusatzstoffen versehen und dann mittels einer Tablettenpresse zu Brausetabletten tablettiert.

Die Temperatur, bei welcher das erfindungsgemässe Verfahren durchgeführt wird, ist nicht kritisch und liegt am besten zwischen 40 und 80°C. Der Unterdruck, bei dem gearbeitet wird, soll möglichst niedrig sein. So kann das Anfangsteilvakuum einen Druck von zum Beispiel 10mbar aufweisen. Das polare Lösungsmittel ist vorzugsweise Wasser, das in einer Menge von 0.2 bis 2 Masse-%, bezogen auf das Gewicht des zu behandelnden Gemisches, eingesetzt wird.

Bei der Durchführung des erfindungsgemässen Verfahrens hat es sich gezeigt, dass bei Anwendung geeigneter Konzentrationen der Reaktionspartner und geeigneter Mengen an polarem Lösungsmittel nur ein Teil der Reaktionspartner reagiert, und dass alsbald, nach Ablauf von 10-20 Minuten, die Reaktion deutlich langsamer wird. Wird nun das so zur Reaktion gebrachte und langsam weiter gerührte Gemisch schlagartig getrocknet, dann kann dieser Vorgang unter geeigneten Bedingungen wiederholt werden, wobei es sich bei der Wiederholung herausstellt, dass der genannte Vorgang verlangsamt ist, weil ein grosser Teil der Oberfläche der Säure bereits durch Alkali oder Erdalkalisalze passiviert und reaktionsträge gemacht wurde. Wenn nun dieselbe Reaktion ein drittes Mal durchgeführt wird, so kann man feststellen, dass fast keine Reaktion mehr stattfindet, da die gesamte Oberfläche bereits passiviert ist und die einzelnen Reaktionspartner in sich abgepuffert sind. Es ist also eine reaktionsträge Mischung entstanden, die selbst in Berührung mit geringen Mengen an polaren Lösungsmitteln nicht mehr oder nur sehr träge reagiert. Es liegt auf der Hand, dass die hergestellten Mischungen im trockenen Zustand eine sehr hohe Stabilität auch bei höheren Temperaturen aufweisen, sodass der Zutritt von Luftfeuchtigkeit oder eine lange Lagerung bei höheren Temperaturen keine weiteren Reaktionen hervorrufen können.

Da erfindungsgemäss die freigesetzte Menge CO₂ festgestellt wird, kann man im Zuge einfacher stöchiometrischer Berechnungen ermitteln, wieviel Bicarbonat verbraucht bzw. wieviel Salz mit der Lactobionsäure gebildet wurde. Man hat es nun in der Hand, durch geeignete Parameter wie etwa die Rührgeschwindigkeit, die eingesetzte Feuchtigkeitsmenge von zum Beispiel Wasser und Alkohol, die Korngrösse der eingesetzten Lactobionsäure zu bestimmen, und wieviel Lactobionsäure an der Oberfläche derselben zum Säuresalz umgesetzt wurde.

Bringt man beispielsweise in einem verschlossenen 100 l Gefäss 40kg einer Reaktionsmischung zur Reaktion, dann wird der freie Raum zwischen Masse und Kesselinhalt je nach Schüttgewicht der Masse etwa 50 l betragen. Hat man das Gefäss vor der Reaktion evakuiert, dann werden diese 50 l freier Raum praktisch gasfrei sein. Lasst man nun eine mengenmässig bestimmte Menge Feuchtigkeit unter gleichzeitigem Rühren in die Masse einströmen, dann wird das entsprechende Kohlendioxyd den überstehenden Raum von 50 Litern ausfüllen. Diese Befüllung des Raumes lässt sich leicht mit einem guten Manometer überprüfen. Hat also das Anfangsteilvakuum 10mbar betragen und ist durch die Reaktion der Druck im Vakuumkessel auf zum Beispiel Atmosphärendruck gestiegen, dann bedeutet das, dass etwa 50 l Kohlendioxyd erzeugt wurden.

Es lässt sich nun zurückrechnen, wieviel Lactobionsäure in diesem Falle verbraucht bzw. wieviel Lactobionat bzw, wieviel Citrat hergestellt wurde. Die mehrmalige Wiederholung der beschriebenen Vakuumbehandlung führt fast zum Stillstand der Reaktion zwischen den Reaktionspartnern, also der Lactobionsäure und dem Calciumsalz des Calciums.

Auch bei Gegenwart polarer Lösungsmittel und trotz der geringen Mengen an umgesetzten Materialen lässt sich kaum mehr eine Reaktion feststellen, sodass nach endgültiger Trocknung des Produktes eine ausserordentlich widerstandsfähige, aber immer noch reaktionsfähige Masse erhalten wird. Die Ergebnisse sind bei Gleichhaltung der vorgenannten Parameter überdies streng reproduzierbar und gewährleisten die Gewinnung eines Produktes von immer gleichbleibender Qualität.

Als theoretische Erklärung für den durch das erfindungsgemässe Verfahren erzielten Effekt kann vorgebracht werden, dass die Berührungsflächen der Reaktionspartner eine Pufferzone bilden, die durch verschiedene Alkali- oder Erdalkalisalze der entsprechenden Säure gebildet werden. Diese Pufferzonen werden sich selbstverständlich je nach der Oberfläche der einzelnen Kristalle und nach der Reaktionsfähigkeit ausbilden.

Nachfolgend wird ein Beispiel mit Zahlen angegeben: In einem 100 Liter Vakuumkessel werden 29 kg Zitronensäure und 1kg bis 3kg Lactobionsäure angesetzt und unter Rühren 5 Minuten lang auf 60°C erhitzt, wonach zur Kontrolle der Restfeuchtigkeit kurz auf 20mbar Druck im Vakuumkessel evakuiert wird. Nach dem Aufheben des Vakuums werden 10 kg Calciumcarbonat hinzugefügt, worauf unter weiterem Rühren abermals auf 60°C erhitzt wird. Dieses Gemisch mit der Lactobionsäure hat dann ein Schüttgewicht pro Volumen von annähernd 1250 kg/m³, sodass der im Vakuumkessel verbleibende Raum etwa 50 Liter beträgt. Dann wird auf 10 mbar evakuiert, von der Pumpe mittels Ventil abgesperrt, und unter Rühren wird eine Menge von 210 ml Wasser eingebracht. Bei der nun einsetzenden Reaktion zwischen der Lactobionsäure und dem Hydrogencarbonat unter Bildung einer passivierenden Oberflächenschicht auf den Säurekristallen und Entwicklung von CO₂ steigt der Druck im Vakuumkessel von 10 mbar auf 1000 mbar an, was einem Volumen an freigesetztem CO₂ von 50 Litern bzw. bei einem Molvolumen des CO₂ bei 60 °C von 27,1 Liter einer Masse an freigesetztem CO₂ von 81 Gramm entspricht.

Daraus resultiert, dass bei dieser ersten Vakuumbehandlung ein Teil der Lactobionsäure mit der Oberflächenschicht der Partikel, das heisst des Calciumsalzes des Calciums reagierte und CO₂ freisetzte. Die Reaktionszeit beträgt ca. 4 min. Jetzt wird das behandelnde Gut getrocknet und vakuumbehandelt, unter denselben Bedingungen und unter Einsatz von 300 ml Wasser wiederholt. Dabei lässt man den Druck im Vakuumkessel wieder auf 1000 mbar ansteigen, entsprechend einem Volumen von 50 Litern bzw. einer Masse von 81 Gramm CO₂. Aufgrund der in der ersten Behandlung erzielten teilweisen Anbrausung der Oberflächenschicht durch die Lactobionsäure ist diesmal die Reaktionszeit schon wesentlich länger und beträgt etwa 10 bis 30 Minuten. Das behandelte Gut wird wieder getrocknet und einer dritten Vakuumbehandlung unterzogen, wobei nur noch ein unbedeutender Druckanstieg gemessen wird, weil die Lactobionsäure aufgebraucht ist, sodass im wesentlichen keine CO₂-Erzeugung mehr stattfindet. Die Oberfläche der Säurekristalle ist weitgehend angebraust bzw. wurde passiviert, das heisst es passiert keine weitere Reaktion. Das Produkt wird dann getrocknet und die erhaltenen Agglomerate können zur gewünschten Teilchengrösse zerkleinert werden und können mit den gewünschten Zusätzen wie Aromastoffe, Vitamine, Süssstoffe und dergleichen versetzt werden. Für das spätere Brausen wird eine Säure in herkömmlicher Art zugegeben, vorzugsweise Zitronensäure, wobei aber auch andere Säuren wie Apfelsäure, Bernsteinsäure, Fumarsäure, Weinsäure oder Ascorbinsäure wie auch Mischungen dieser Säuren funktionieren. Dieses Brausegranulat kann gegebenenfalls in bekannter Weise mittels Tablettenpressen zu Brausetabletten verpresst werden.

Das auf diese Weise erhaltene Produkt hat auch bei tropischen Temperaturen eine ausgezeichnete Lagerfähigkeit über lange Zeitperioden. Das Brausen wird durch die beschriebene Vorbehandlung mittels Lactobionsäure beschleunigt und tiefenwirksam, weil alle Pulverpartikel "angebraust" sind, das heisst sie bieten eine ungleich grössere Oberfläche zur späteren Reaktion mit der beigemengten Säure, zum Beispiel Zitronensäure, sobald die Brausetablette mit Wasser in Berührung kommt.

Legt man eine solchermassen hergestellte Brausetablette auf Raumtemperatur ins Wasser, so dringt dieses rasch in die Oberflächenschicht der Pulverpartikel ein und führt zu einem Brauseeffekt, welcher die ganze Brausetablette gegen das Innere fortschreitend rasch verbraust und vollständig in Wasser löst. Nach weniger als 3 Minuten ist die Brausetablette komplett aufgelöst und die Lösung ist sogar klar.

## Patentansprüche

1. Verfahren zum Herstellen eines Natrium-Ion-freien X-Carbonat oder Hydrogencarbonat Brausepulvers oder -granulats oder einer Natrium-Ion-freien X-Carbonat oder Hydrogencarbonat Brausetablette, bei welchem die Wirksubstanzen, entsprechend wenigstens Calcium-Carbonat, MagnesiumCarbonat, Magnesiumhydrogen-Carbonat, Kalium-Carbonat und Kaliumhydrogen-Carbonat oder Mischungen davon mit wenig Alkohol und Wasser gefeuchtet als Pulver in einem Vakuumkessel bei ständigem Abpumpen in einem Teilvakuum mit wenig Lactobionsäure vermischt werden, wodurch das Calciumsalz des Calciums in der Oberflächenschicht der Pulverpartikel mit der Lactobionsäure reagiert, verbraust und wenig CO₂ freisetzt, wodurch der Druck im Vakuumtopf ansteigt und nach Ende der Brause-Reaktion durch das ständige Abpumpen der Druck im Teilvakuum wieder auf den Anfangswert absinkt und dabei das Pulver austrocknet und ihm nach Entnahme aus dem Vakuumtopf eine Säure zum Brausen als Brausemittel zugegeben wird und es hernach als Brausepulver verwendbar ist oder zu Tabletten verpresst wird.

2. Verfahren zum Herstellen eines Natrium-Ion-freien X-Carbonat oder Hydrocarbonat Brausepulvers oder -granulats oder einer Natrium-Ion-freien X-Carbonat oder Hydrocarbonat Brausetablette nach Anspruch 1, **dadurch gekennzeichnet, dass** als X Calcium, Magnesium oder Kalium oder eine Mischung aus diesen Stoffen eingesetzt wird, und als Säure zum Brausen eine oder mehrere der folgenden eingesetzt wird: Zitronensäure, Apfelsäure, Bernsteinsäure, Fumarsäure, Weinsäure, Ascorbinsäure.

3. Verfahren zum Herstellen eines Natrium-Ion-freien X-Carbonat oder Hydrocarbonat Brausepulvers oder -granulats oder einer Natrium-Ion-freien X-Carbonat oder Hydrocarbonat Brausetablette nach einem der vorangehenden Ansprüche, **dadurch gekennzeichnet, dass** ausserdem Kaliumhydrogencarbonat zugegeben wird, das mit der Lactobionsäure braust und initiales CO₂ erzeugt, zum Starten und Verbessern der Wasserlöslichkeit des Calcium-Carbonates.

4. Verfahren zum Herstellen eines Natrium-Ion-freien X-Carbonat oder Hydrocarbonat Brausepulvers oder -granulats oder einer Natrium-Ion-freien X-Carbonat oder Hydrocarbonat Brausetablette nach einem der vorhergehenden Ansprüche, **dadurch gekennzeichnet, dass** die Zugabe von Lactobionsäure im Teilvakuum mehrmals erfolgt, wonach jedes Mal der Druck im Vakuumkessel langsamer ansteigt, bis die Oberflächenschicht des Calcium-Carbonates vollständig passiviert ist, und dann das vakuumgetrocknete Pulver dem Vakuumkessel entnommen und weiterverarbeitet wird.

5. Natrium-Ion-freies X-Carbonat oder Hydrogencarbonat Brausepulver oder - granulat oder Natrium-Ion-freie X-Carbonat oder Hydrocarbonat Brausetablette, hergestellt nach dem Verfahren nach einem der Ansprüche 1 bis 4, enthaltend wenigstens ein X-Carbonat sowie eine Säure als Brausemittel, und wobei die verpressbaren Pulverpartikel oder die zu einer Brausetablette verpressten Partikel mittels Lactobionsäure in ihrer Oberflächenschicht angebraust sind.

6. Natrium-Ion-freies X-Carbonat oder Hydrocarbonat Brausepulver oder - granulat oder Natrium-Ion-freie X-Carbonat oder Hydrocarbonat Brausetablette, hergestellt nach dem Verfahren einem der Ansprüche 1 bis 4, wobei X beim Carbonat eine der folgenden Stoffe ist: Calcium, Magnesium oder Kalium oder eine Mischung aus diesen Stoffen, und die Säure als Brausemittel zum Brausen eine oder mehrere der folgenden ist: Zitronensäure, Apfelsäure, Braunsteinsäure, Fumarsäure, Weinsäure, Ascorbinsäure, und wobei die verpressbaren Pulverpartikel oder die zu einer Brausetablette verpressten Partikel mittels Lactobionsäure in ihrer Oberflächenschicht angebraust sind.

7. Natrium-Ion-freies X-Carbonat oder Hydrocarbonat Brausepulver oder - granulat oder Natrium-Ion-freie X-Carbonat oder Hydrocarbonat Brausetablette, hergestellt nach dem Verfahren einem der Ansprüche 1 bis 4, enthaltend wenigstens Calcium-Carbonat und Kaliumhydrogencarbonat, sowie Zitronensäure als Brausemittel, und wobei die verpressbaren Pulverpartikel oder die zu einer Brausetablette verpressten Partikel mittels Lactobionsäure in ihrer Oberflächenschicht angebraust sind.

8. Natrium-Ion-freies X-Carbonat oder Hydrocarbonat Brausepulver oder - granulat oder Natrium-Ion-freie X-Carbonat oder Hydrocarbonat Brausetablette nach Anspruch 5, enthaltend nebst optionalen weiteren Substanzen wenigstens Calcium-Carbonat sowie Calcium-Ion, und anstelle von Natrium-Ion für das Löslichmachen der Calcium-Carbonat (CaCOs)-Pulveroberfläche einzig Lactobionsäure, sowie Zitronensäure für das Anbrausen beim Lösen des Brausepulvers oder der Brausetablette in Wasser.
